# EUROPEAN PATENT APPLICATION

(11) **EP 4 324 474 A1**
(43) Date of publication of application: **21.02.2024**
(21) Application number: 22787476.5
(22) Date of filing: 11.04.2022
(51) Int. Cl.: A61K 38/07, C07K 5/107, A61P 17/04

(54) **USE OF PEPTIDE AMIDE COMPOUND IN PREPARATION OF DRUG FOR TREATING PRURITUS**

(30) Priority: 12.04.2021 CN 202110386787; 10.09.2021 CN 202111056972; 22.12.2021 CN 202111569320
(71) Applicant: Xizang Haisco Pharmaceutical Co., Ltd., Lhoka, Tibet 856099 (CN)
(72) Inventor: WANG, Yongrui, Chengdu, Sichuan 611130 (CN); ZHANG, Zizhen, Chengdu, Sichuan 611130 (CN); ZHANG, Chunnan, Chengdu, Sichuan 611130 (CN); GU, Xue, Chengdu, Sichuan 611130 (CN); CHU, Chang, Chengdu, Sichuan 611130 (CN); GOU, Xiaoli, Chengdu, Sichuan 611130 (CN)
(74) Representative: Hoffmann Eitle
(86) International application number: PCT/CN2022/086066
(87) International publication number: WO 2022/218250

(57) **Abstract**

Use of a compound represented by formula (I) or a stereoisomer, hydrate, metabolite, solvate, pharmaceutically acceptable salt, eutectic, or a composition thereof in preparation of a drug for treating pruritus. Also provided is a method for treating pruritus, the method comprises administering an effective dose of the compound represented by formula (I) or the stereoisomer, hydrate, metabolite, solvate, pharmaceutically acceptable salt, eutectic, or a composition thereof.

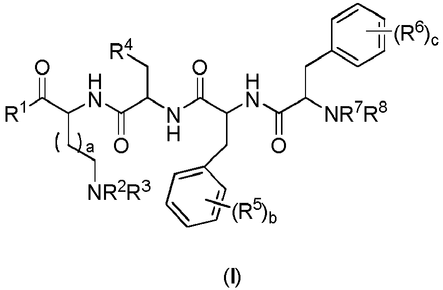

## Description

### Technical Field

The present invention relates to the use of a compound represented by formula (I) or a stereoisomer, hydrate, metabolite, solvate, pharmaceutically acceptable salt or eutectic thereof, and a composition thereof in the preparation of a drug for treating pruritus.

### Background Art

Pruritus or itching is a sensation that triggers the desire to scratch. Pruritus lasting more than 6 weeks is usually defined as chronic pruritus. The presence of pruritus not only interferes with the patient's emotion, but repeated scratching on the pruritus site can also lead to local skin damage and infection, which seriously hinders the patient's normal life. Pruritus can be classified in a variety of ways according to the sites of origin, pathogenesis, and skin lesion manifestation of pruritus, such as pruritus that originates from the skin, systemic pruritus, neurogenic pruritus and pruritus caused by mental disorder (HAO, Fei, Guidelines for Management of Chronic Pruritus (Edition 2018)).

At present, the epidemiological data of pruritus are relatively limited, and there is still a lack of relevant data in China. There are foreign reports indicating that the prevalence of chronic pruritus in adults reaches 10% or more, while that in the elderly is as high as 60%. Among disease states, the prevalence of chronic pruritus varies greatly. For example, pruritus occurs in almost 100% of patients with atopic dermatitis and urticaria, approximately 80% of patients with psoriasis, approximately 80%-100% of patients with primary biliary cholangitis, and approximately 40%-70% of patients with chronic kidney disease (HAO, Fei, Guidelines for Management of Chronic Pruritus (Edition 2018)).

Although there are currently various drugs with different mechanisms for chronic pruritus, they usually have more adverse reactions or can only meet the treatment needs of certain chronic pruritus. Therefore, there is currently still a large unmet clinical need for the treatment of chronic pruritus.

The treatment of chronic pruritus usually involves the following steps in order: eliminating aetiology or treating underlying diseases; using a topical humectant, an oral antihistamine and a topical glucocorticoid; and in view of the mechanism of pruritus, systemically administrating a hormone or non-hormonal anti-inflammatory preparation, a central nervous system depressant, a drug antagonizing pruritus mediators, a biological preparation, etc. (HAO, Fei, Guidelines for Management of Chronic Pruritus (Edition 2018)).

Antihistamines are one of the most commonly used systemic antipruritic drugs, but they are usually used for diseases related to increased mast cell degranulation such as urticaria, and have no definite effect on certain chronic pruritus. The general use of antihistamines in the treatment of non-histamine-mediated pruritus, such as AD and psoriasis, is not recommended in European guidelines. There is no evidence indicating that antihistamines are effective for pruritus caused by some systemic diseases, including chronic renal failure, cholestatic liver disease, blood system diseases, thyroid disease, etc. (Elke WEISSHAAR, European S2k Guideline on Chronic Pruritus). Systematic administration of hormones in the treatment of pruritus caused by inflammatory skin diseases has a rapid onset of action, but should only be used for a short period of time due to numerous adverse reactions. Gabapentin and pregabalin as antiepileptic drugs have a certain effect on neuropathic pruritus and pruritus related to chronic kidney disease. However, a large national cohort analysis based on the United States Renal Data System showed that the administration of the above-mentioned drugs may lead to a significantly increased risk of altered mental status, fall down, and fracture (Ishida JH, et al. J Am Soc Nephrol. 2018 Jul; 29(7): 1970-1978). Antidepressants can exert antipruritic effects by acting on serotonin, histamine, etc. However, adverse reactions caused by antidepressants are more common, such as mental disorders, lethargy, irritability, dry mouth, aggravated pruritus, atrioventricular block, even death, etc., especially in the elderly. In addition, selective serotonin reuptake inhibitors (SSRIs) generally have a slower onset of action, starting in approximately 2-3 weeks (Elke WEIS SHAAR, European S2k Guideline on Chronic Pruritus). Immunosuppressants, such as cyclosporine, azathioprine and methotrexate, can be used for pruritus caused by inflammatory skin diseases and chronic prurigo, and the main adverse reaction thereof is immunosuppression, including leukopenia, impaired liver and kidney function, etc. At present, the biological preparations are also developing rapidly. Dupilumab is recommended for patients with atopic dermatitis, and omalizumab is recommended for patients with chronic spontaneous urticaria (Elke WEISSHAAR, European S2k Guideline on Chronic Pruritus).

In addition, studies have confirmed that endogenous or exogenous endorphins are involved in the occurrence of pruritus, and the administration of opioid receptor antagonists or κ receptor agonists can effectively inhibit pruritus. Opioid receptor antagonists include naloxone, naltrexone, etc., but adverse reactions caused thereby are common, including increased or decreased blood pressure, tachycardia, liver damage, rash, etc. Early κ receptor agonists are mostly non-selective and mainly used for acute pruritus caused by morphine (Phan, N.Q., et al. Acta Derm Venereol, 2012. 92(5): p. 555-60.). Nalfurafine, a selective κ receptor agonist, has been approved in Japan and Korea for the treatment of pruritus in haemodialysis patients and patients with chronic liver disease. However, nalfurafine can enter the central nervous system, thereby resulting in central nervous system-related side effects, such as insomnia, lethargy, dizziness, and constipation. Therefore, there is a need for the development of a new peripherally-selective κ receptor agonist, such that the peripheral κ receptor agonist only acts on the periphery and rarely passes through the blood-brain barrier, and can maintain the high agonistic activity on κ receptors, while the inherent side effects of traditional central nervous system-selective κ receptor agonists are also avoided.

### Summary of the Invention

The present invention provides the use of a compound represented by formula (I) or a stereoisomer, hydrate, metabolite, solvate, pharmaceutically acceptable salt or eutectic thereof, and a composition thereof in the preparation of a drug for treating pruritus, characterized in that the compound represented by formula (I) or the stereoisomer, hydrate, metabolite, solvate, pharmaceutically acceptable salt, eutectic or composition thereof is administered in an effective dose to a mammal, 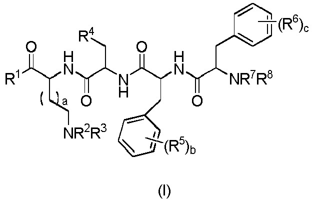 wherein
R₁ is selected from 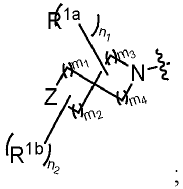
m1, m2, m3 and m4 are each independently selected from 0, 1, 2, 3 or 4; m1 and m2 are not both 0; m3 and m4 are not both 0;
n1 and n2 are each independently selected from 0, 1, 2, 3 or 4;
Z is selected from CR^{z1}R^{z2} or NR^{z3};
R^{z1} and R^{z2} are each independently selected from H, F, Cl, Br, I, OH, CF₃, nitro, C₁₋₆ alkyl, C₁₋₆ alkoxy, C₂₋₆ alkenyl, C₂₋₆ alkynyl, -C(=O)-C₁₋₆ alkyl, -(CH₂)q-C(=O)O-C₁₋₆ alkyl, -(CH₂)q-NR^{1e}R^{1f}, -(CH₂)q-COOH, -(CH₂)q-CONH₂, C₃₋₈ carbocyclyl, or 3- to 8-membered heterocyclyl, wherein the alkyl, alkoxy, alkenyl, alkynyl, carbocyclyl or heterocyclyl is optionally further substituted with 0-5 substituents selected from F, Cl, Br, I, OH, CF₃, =O, carboxyl, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, the heterocyclyl contains 1 to 3 heteroatoms optionally selected from N, O or S, and when the heteroatom is selected from S, the heterocyclyl is optionally further S, S=O or S(=O)₂;
R^{1e} and R^{1f} are each independently selected from H, C₁₋₆ alkyl, -C(=O)O-C₁₋₆ alkyl, -C(=O)O-(CH₂)q-C₃₋₈ carbocyclyl or -C(=O)O-(CH₂)q-3- to 8-membered heterocyclyl, wherein the alkyl, carbocyclyl or heterocyclyl is optionally further substituted with 0-5 substituents selected from F, Cl, Br, I, OH, CF₃, cyano, nitro, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
or R^{z1} and R^{z2} together with the carbon atom to which they are attached form a 3- to 10-membered nitrogen-containing heterocyclic ring, wherein the ring is optionally further substituted with a substituent selected from F, Cl, Br, I, OH, CF₃, cyano, nitro, =O, C₁₋₆ alkyl, C₁₋₆ alkoxy, C₂₋₆ alkenyl, C₂₋₆ alkynyl, C.carbocyclyl or 3- to 8-membered heterocyclyl;
R^{1a} and R^{1b} are each independently selected from F, CF₃, NH₂, C₁₋₆ alkyl, C₂₋₆ alkenyl, C₂₋₆ alkynyl or 3- to 8-membered heterocyclyl, wherein the alkyl, alkenyl, alkynyl or heterocyclyl is optionally further substituted with 0-5 substituents selected from F, Cl, Br, I, OH, CF₃, nitro, cyano, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{z3} is independently selected from H, -C(=O)-C₁₋₆ alkyl, -C(=O)O-C₁₋₆ alkyl, - C(=O)-C₃₋₈ carbocyclyl, -C(=O)O-C₃₋₈ carbocyclyl, -C(=O)O-(3- to 8-membered heterocyclyl), -S(=O)p-C₁₋₆ alkyl, -S(=O)p-C₃₋₈ carbocyclyl, -S(=O)p-(3- to 8-membered heterocyclyl), -C(=O)NR^{1g}R^{1h}, -S(=O)p-NR¹ⁱR^{1j} or 3- to 8-membered heterocyclyl, wherein the alkyl, carbocyclyl or heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{1g}, R^{1h}, R^{1i'} and R^{1j} are each independently selected from H or C₁₋₆ alkyl;
or R^{1g} and R^{1h} together with the nitrogen atom to which they are attached form a 3- to 10-membered heterocyclic ring, wherein the ring is optionally further substituted with a substituent selected from F, Cl, Br, I, OH, CF₃, nitro, cyano, amino, C₁₋₆ alkyl, C₁₋₆ alkoxy, C₂₋₆ alkenyl, C₂₋₆ alkynyl or -S(=O)p-C₁₋₆ alkyl, and the heterocyclyl contains 1 to 3 heteroatoms selected from N, O or S;
q is selected from 0, 1, 2, 3 or 4;
p is selected from 0, 1 or 2;
a is selected from 0, 1, 2 or 3;
R⁴ is independently selected from H, C₁₋₆ alkyl, C₂₋₆ alkenyl, C₂₋₆ alkynyl or - (CH₂)q-C₃₋₈ carbocyclyl, wherein the alkyl, alkenyl, alkynyl or carbocyclyl is optionally further substituted with 0-5 substituents selected from F, Cl, Br, I, OH, CN, CF₃, NO₂, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
R², R³, R⁷ and R⁸ are each independently selected from H, C₁₋₆ alkyl, -C(=O)O-C₁₋₄ alkyl, -C(=O)O-(CH₂)q-C₃₋₈ carbocyclyl, -C(=O)O-(CH₂)q-3- to 8-membered heterocyclyl or 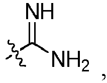 wherein the alkyl, carbocyclyl or heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
b is selected from 0, 1, 2, 3, 4 or 5;
c is selected from 0, 1, 2, 3, 4 or 5;
R⁵ and R⁶ are each independently selected from F, Cl, Br, I, OH, CN, CF₃, cyano, nitro, C₁₋₄ alkyl, -OR^{5a}, -C(O)OR^{5b}, -SR^{5c}, -S(O)R^{5d}, -S(O)₂R^{5e} or -NR^{5f}R^{5g};
R^{5a}, R^{5b}, R^{5c}, R^{5d}, R^{5e}, R^{5f} and R^{5g} are each independently selected from H or C₁₋₄ alkyl;
or R^{5f} and R^{5g} together with the nitrogen atom to which they are attached form a 5- to 6-membered heterocyclic ring, wherein the heterocyclyl contains 1 to 3 heteroatoms optionally selected from N, O or S.

The present invention relates to a method for treating pruritus, comprising administering an effective dose of the compound represented by formula (I) or the stereoisomer, hydrate, metabolite, solvate, pharmaceutically acceptable salt, eutectic or composition thereof to a mammal, 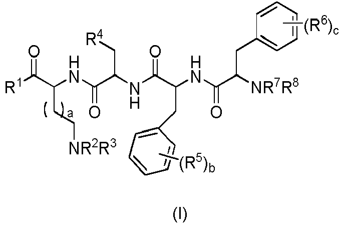 wherein
R₁ is selected from 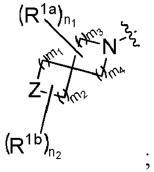
m1, m2, m3 and m4 are each independently selected from 0, 1, 2, 3 or 4; m1 and m2 are not both 0; m3 and m4 are not both 0;
n1 and n2 are each independently selected from 0, 1, 2, 3 or 4;
Z is selected from CR^{z1}R^{z2} or NR^{z3};
R^{z1} and R^{z2} are each independently selected from H, F, Cl, Br, I, OH, CF₃, nitro, C₁₋₆ alkyl, C₁₋₆ alkoxy, C₂₋₆ alkenyl, C₂₋₆ alkynyl, -C(=O)-C₁₋₆ alkyl, -(CH₂)q-C(=O)O-C₁₋₆ alkyl, -(CH₂)q-NR^{1e}R^{1f}, -(CH₂)q-COOH, -(CH₂)q-CONH₂, C₃₋₈ carbocyclyl, or 3- to 8-membered heterocyclyl, wherein the alkyl, alkoxy, alkenyl, alkynyl, carbocyclyl or heterocyclyl is optionally further substituted with 0-5 substituents selected from F, Cl, Br, I, OH, CF₃, =O, carboxyl, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, the heterocyclyl contains 1 to 3 heteroatoms optionally selected from N, O or S, and when the heteroatom is selected from S, the heterocyclyl is optionally further S, S=O or S(=O)₂;
R^{1e} and R^{1f} are each independently selected from H, C₁₋₆ alkyl, -C(=O)O-C₁₋₆ alkyl, -C(=O)O-(CH₂)q-C₃₋₈ carbocyclyl or -C(=O)O-(CH₂)q-3- to 8-membered heterocyclyl, wherein the alkyl, carbocyclyl or heterocyclyl is optionally further substituted with 0-5 substituents selected from F, Cl, Br, I, OH, CF₃, cyano, nitro, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
or R^{z1} and R^{z2} together with the carbon atom to which they are attached form a 3- to 10-membered nitrogen-containing heterocyclic ring, wherein the ring is optionally further substituted with a substituent selected from F, Cl, Br, I, OH, CF₃, cyano, nitro, =O, C₁₋₆ alkyl, C₁₋₆ alkoxy, C₂₋₆ alkenyl, C₂₋₆ alkynyl, C.carbocyclyl or 3- to 8-membered heterocyclyl;
R^{1a} and R^{1b} are each independently selected from F, CF₃, NH₂, C₁₋₆ alkyl, C₂₋₆ alkenyl, C₂₋₆ alkynyl or 3- to 8-membered heterocyclyl, wherein the alkyl, alkenyl, alkynyl or heterocyclyl is optionally further substituted with 0-5 substituents selected from F, Cl, Br, I, OH, CF₃, nitro, cyano, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{z3} is independently selected from H, -C(=O)-C₁₋₆ alkyl, -C(=O)O-C₁₋₆ alkyl, - C(=O)-C₃₋₈ carbocyclyl, -C(=O)O-C₃₋₈ carbocyclyl, -C(=O)O-(3- to 8-membered heterocyclyl), -S(=O)p-C₁₋₆ alkyl, -S(=O)p-C₃₋₈ carbocyclyl, -S(=O)p-(3- to 8-membered heterocyclyl), -C(=O)NR^{1g}R^{1h}, -S(=O)p-NR¹ⁱR^{1j} or 3- to 8-membered heterocyclyl, wherein the alkyl, carbocyclyl or heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{1g}, R^{1h}, R^{1i'} and R^{1j} are each independently selected from H or C₁₋₆ alkyl;
or R^{1g} and R^{1h} together with the nitrogen atom to which they are attached form a 3- to 10-membered heterocyclic ring, wherein the ring is optionally further substituted with a substituent selected from F, Cl, Br, I, OH, CF₃, nitro, cyano, amino, C₁₋₆ alkyl, C₁₋₆ alkoxy, C₂₋₆ alkenyl, C₂₋₆ alkynyl or -S(=O)p-C₁₋₆ alkyl, and the heterocyclyl contains 1 to 3 heteroatoms selected from N, O or S;
q is selected from 0, 1, 2, 3 or 4;
p is selected from 0, 1 or 2;
a is selected from 0, 1, 2 or 3;
R⁴ is independently selected from H, C₁₋₆ alkyl, C₂₋₆ alkenyl, C₂₋₆ alkynyl or - (CH₂)q-C₃₋₈ carbocyclyl, wherein the alkyl, alkenyl, alkynyl or carbocyclyl is optionally further substituted with 0-5 substituents selected from F, Cl, Br, I, OH, CN, CF₃, NO₂, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
R², R³, R⁷ and R⁸ are each independently selected from H, C₁₋₆ alkyl, -C(=O)O-C₁₋₄ alkyl, -C(=O)O-(CH₂)q-C₃₋₈ carbocyclyl, -C(=O)O-(CH₂)q-3- to 8-membered heterocyclyl or 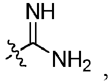 wherein the alkyl, carbocyclyl or heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
b is selected from 0, 1, 2, 3, 4 or 5;
c is selected from 0, 1, 2, 3, 4 or 5;
R⁵ and R⁶ are each independently selected from F, Cl, Br, I, OH, CN, CF₃, cyano, nitro, C₁₋₄ alkyl, -OR^{5a}, -C(O)OR^{5b}, -SR^{5c}, -S(O)R^{5d}, -S(O)₂R^{5e} or -NR^{5f}R^{5g};
R^{5a}, R^{5b}, R^{5c}, R^{5d}, R^{5e}, R^{5f} and R^{5g} are each independently selected from H or C₁₋₄ alkyl;
or R^{5f} and R^{5g} together with the nitrogen atom to which they are attached form a 5- to 6-membered heterocyclic ring, wherein the heterocyclyl contains 1 to 3 heteroatoms optionally selected from N, O or S. In some embodiments of the present invention which relate to the use in a drug for preventing or treating pruritus and the method for treating pruritus, the compound represented by formula (I) is selected from a compound represented by formula (II): 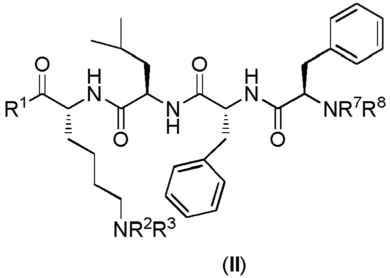 wherein
   R₁ is selected from 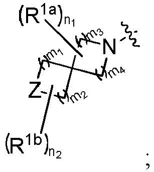
   m1, m2, m3 and m4 are each independently selected from 0, 1 or 2; m1 and m2 are not both 0; m3 and m4 are not both 0;
   n1 and n2 are each independently selected from 0 or 2;
   Z is selected from CR^{z1}R^{z2} or NR^{z3};
   R^{1a} and R^{1b} are independently selected from F or NH₂;
   R^{z1} and R^{z2} are each independently selected from H, carboxyl, 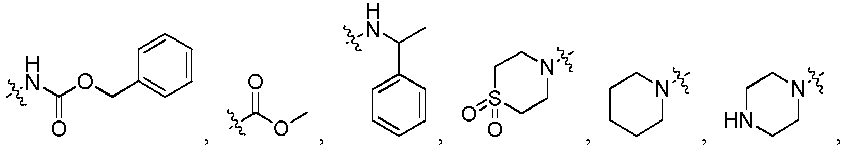 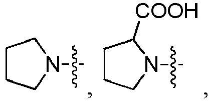 , amino, -CH₂NH₂ or 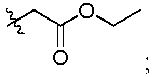
   or R^{z1} and R^{z2} can together with the carbon atom to which they are attached form a lactam 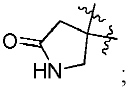
   R^{z3} is independently selected from H, -C(=O)-C₁₋₄ alkyl, -C(=O)O-C₁₋₄ alkyl, - C(=O)-C₃₋₆ carbocyclyl, -C(=O)O-C₃₋₆ carbocyclyl, -S(=O)p-C₁₋₄ alkyl, -S(=O)p-C₃₋₆ carbocyclyl, -C(=O)NR^{1g}R^{1h}, -S(=O)p-NR¹ⁱR^{1j} or 3- to 6-membered heterocyclyl, wherein the alkyl, carbocyclyl or heterocyclyl is optionally further substituted with 0-5 substituents selected from F, Cl, Br, I, OH, CF₃, nitro, cyano, amino, methyl, ethyl, methoxy, ethoxy, cyclopropyl or phenyl, and the heterocyclyl contains 1 to 3 heteroatoms optionally selected from N, O or S;
   R^{1g}, R^{1h}, R^{1i'} and R^{1j} are each independently selected from H or C₁₋₄ alkyl;
   or R^{1g} and R^{1h} together with the nitrogen atom to which they are attached form a 4- to 6-membered heterocyclic ring, wherein the ring is optionally further substituted with a substituent selected from F, CF₃, methyl, methoxy or -S(=O)p-C₁₋₄ alkyl, and the heterocyclyl contains 1 to 3 heteroatoms selected from N, O or S;
   p is selected from 2;
   R², R³, R⁷ and R⁸ are each independently selected from H, methyl or C(=O)O-tert-butyl.

In some embodiments of the present invention which relate to the use in a drug for preventing or treating pruritus and the method for treating pruritus, the compound represented by formula (II) is selected from a compound represented by structural formula (A) as follows:

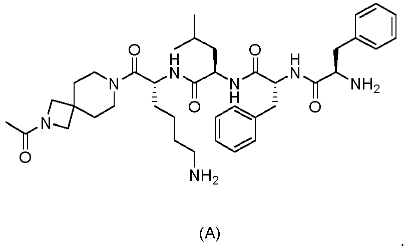

In some embodiments of the present invention which relate to the use in a drug for preventing or treating pruritus and the method for treating pruritus, an effective dose of the compound represented by structural formula (A) or a stereoisomer, hydrate, metabolite, solvate, pharmaceutically acceptable salt, eutectic or composition thereof is administered to a mammal.

In some embodiments of the present invention which relate to the use in a drug for preventing or treating pruritus and the method for treating pruritus, the pharmaceutically acceptable salt is selected from propionate, mesylate, acetate, citrate, D-tartrate, besylate, phosphate, aspartate, L-tartrate, maleate, fumarate, benzoate, lactate, hydrochloride, formate, hydrobromide, sulphate, nitrate, phosphate, trifluoroacetate, succinate, mandelate, malonate, malate, 2-hydroxypropionate, oxalate, glycolate, salicylate, a citric acid salt, glutamate, cinnamate, p-toluenesulfonate, benzenesulfonate, ethanesulfonate or triflate.

In some embodiments of the present invention which relate to the use in a drug for preventing or treating pruritus and the method for treating pruritus, the pruritus is selected from pruritus caused by a disease, pruritus caused by a drug, or kidney disease-related pruritus.

In some embodiments of the present invention which relate to the use in a drug for preventing or treating pruritus and the method for treating pruritus, the pruritus is selected from kidney disease-related pruritus in haemodialysis patients.

In some embodiments of the present invention which relate to the use in a drug for preventing or treating pruritus and the method for treating pruritus, the pruritus is selected from skin pruritus caused by haemodialysis.

In some embodiments of the present invention which relate to the use in a drug for preventing or treating pruritus and the method for treating pruritus, the pruritus caused by a disease includes pruritus caused by an inflammatory or non-inflammatory skin disease, a peripheral or systemic disease, etc.

In some embodiments of the present invention which relate to the use in a drug for preventing or treating pruritus and the method for treating pruritus, the inflammatory or non-inflammatory skin disease includes, but is not limited to, atopic dermatitis, neurodermatitis, contact dermatitis, seborrheic dermatitis, prurigo or senile dermatosis.

In some embodiments of the present invention which relate to the use in a drug for preventing or treating pruritus and the method for treating pruritus, the peripheral or systemic disease includes, but is not limited to, a liver disease, a kidney disease, an autoimmune disease or peripheral neuropathy.

In some embodiments of the present invention which relate to the use in a drug for preventing or treating pruritus and the method for treating pruritus, the pruritus caused by a drug includes, but is not limited to pruritus caused by obeticholic acid, an antibiotic, an angiotensin converting enzyme inhibitor, a xanthine oxidase inhibitor or morphine.

In some embodiments of the present invention which relate to the use in a drug for preventing or treating pruritus and the method for treating pruritus, the liver disease includes, but is not limited to, an autoimmune liver disease (e.g., primary biliary cholangitis, primary sclerosing cholangitis or autoimmune hepatitis), viral hepatitis (e.g., hepatitis A, hepatitis B, hepatitis C, hepatitis D or hepatitis E), tumour (e.g., hepatocellular carcinoma or cholangiocarcinoma), liver cirrhosis, alcoholic liver disease, non-alcoholic fatty liver disease, drug-induced hepatitis, cholestasis (e.g., benign recurrent intrahepatic cholestasis, progressive familial intrahepatic cholestasis or intrahepatic cholestasis of pregnancy) or jaundice; the kidney disease includes, but is not limited to, a chronic kidney disease, uraemia or diabetic nephropathy;
the autoimmune disease includes, but is not limited to, scleroderma or Sjogren syndrome;
the peripheral neuropathy includes, but is not limited to, brachioradial pruritus, notalgia paresthetica or small fibre neuropathy.

In some embodiments of the present invention which relate to the use in a drug for preventing or treating pruritus and the method for treating pruritus, an effective dose of the compound represented by structural formula (A) or the stereoisomer, hydrate, metabolite, solvate, pharmaceutically acceptable salt, eutectic or composition thereof is administered to a mammal, and in terms of the compound represented by structural formula (A), the amount of the compound is 1-1000 µg/day.

In some embodiments of the present invention which relate to the use in a drug for preventing or treating pruritus and the method for treating pruritus, in terms of the compound represented by structural formula (A), the amount of the compound is 1-1000 µg/day, 1-900 µg/day, 1-800 µg/day, 1-700 µg/day, 1-600 µg/day, 1-500 µg/day, 1-400 µg/day, 1-300 µg/day, 1-200 µg/day, 1-100 µg/day, 10-1000 µg/day, 10-900 µg/day, 10-800 µg/day, 10-700 µg/day, 10-600 µg/day, 10-500 µg/day, 10-400 µg/day, 10-300 µg/day, 10-200 µg/day, 10-100 µg/day, 20-500 µg/day, 20-400 µg/day, 20-300 µg/day, 20-250 µg/day, 20-200 µg/day, 20-150 µg/day, 30-100 µg/day, 30-150 µg/day, 30-250 µg/day, 30-300 µg/day, 30-400 µg/day, 40-400 µg/day, 50-100 µg/day, 50-200 µg/day, 50-300 µg/day, 50-400 µg/day, 50-500 µg/day or 50-600 µg/day.

In some embodiments of the present invention which relate to the use in a drug for preventing or treating pruritus and the method for treating pruritus, in terms of the compound represented by structural formula (A), the compound is administered via injection in an amount of 0.05 µg/kg-0.80 µg/kg.

In some embodiments of the present invention which relate to the use in a drug for preventing or treating pruritus and the method for treating pruritus, in terms of the compound represented by structural formula (A), the compound is administered via injection in an amount of 0.05 µg/kg, 0.15 µg/kg, 0.30 µg/kg, 0.60 µg/kg or 0.80 µg/kg.

In some embodiments of the present invention which relate to the use in a drug for preventing or treating pruritus and the method for treating pruritus, in terms of the compound represented by structural formula (A), the compound is administered via injection in an amount of 2 µg-240 µg/day.

In some embodiments of the present invention which relate to the use in a drug for preventing or treating pruritus and the method for treating pruritus, in terms of the compound represented by structural formula (A), the compound is administered via injection in an amount of 2 µg-5 µg/day, 6 µg-15 µg/day, 12 µg-30 µg/day, 24 µg-60 µg/day, 32-80 µg/day, 4 µg-10 µg/day, 12 µg-30 µg/day, 24 µg-60 µg/day, 48 µg-120 µg/day, 64-160 µg/day, 6 µg-15 µg/day, 18 µg-45 µg/day, 36 µg-90 µg/day, 72 µg-180 µg/day or 96-240 µg/day.

In some embodiments of the present invention which relate to the use in a drug for preventing or treating pruritus and the method for treating pruritus, the compound represented by structural formula (A) or the stereoisomer, hydrate, metabolite, solvate, pharmaceutically acceptable salt, eutectic or composition thereof is administered at an interval selected from once a day, twice a day, three times a day, once a week, twice a week, three times a week or once every other day.

In some embodiments of the present invention which relate to the use in a drug for preventing or treating pruritus and the method for treating pruritus, the compound represented by structural formula (A) or the stereoisomer, hydrate, metabolite, solvate, pharmaceutically acceptable salt, eutectic or composition thereof is administered via a route selected from oral route, injection, drip, transdermal route, sublingual route, parenteral route, intraperitoneal route, rectal route, buccal route, nasal drip, inhalation, local delivery, subcutaneous route, intra-adipose route, intra-articular route, intraperitoneal route or intrathecal route, preferably oral route, injection or nasal spray.

In some embodiments of the present invention which relate to the use in a drug for preventing or treating pruritus and the method for treating pruritus, the compound represented by structural formula (A) or the stereoisomer, hydrate, metabolite, solvate, pharmaceutically acceptable salt, eutectic or composition thereof is administered via a route selected from oral route, intravenous injection, intravenous drip, intra-arterial injection, intramuscular injection, subcutaneous injection, intra-articular injection, intraperitoneal injection, intrathecal injection or nasal drip, preferably oral route, injection or nasal spray.

Unless stated to the contrary, the terms used in the description and claims have the following meanings.

The "alkyl" refers to a linear or branched monovalent saturated hydrocarbon group, in which the main chain comprises a linear or branched group of 1 to 10 carbon atoms, preferably 1 to 8 carbon atoms, further preferably 1 to 6 carbon atoms, more preferably 1 to 4 carbon atoms, and most preferably 1 to 2 carbon atoms. Examples of alkyl include, but are not limited to methyl, ethyl, n-propyl, isopropyl, n-butyl, isobutyl, sec-butyl, tert-butyl, n-pentyl, 2-pentyl, 3-pentyl, 2-methyl-2-butyl, 3-methyl-2-butyl, n-hexyl, n-heptyl, n-octyl, n-nonyl and n-decyl. The alkyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxylalkyl, C₁₋₆ alkoxy, C₂₋₆ alkenyl, C₂₋₆ alkynyl, C₃₋₈ carbocyclyl, 3- to 8-membered heterocyclyl, -(CH₂) ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}, wherein R¹⁹ and R^{19a} are each independently selected from H, hydroxyl, amino, carboxyl, C₁₋₈ alkyl, C₁₋₈ alkoxy, C₂₋₈ alkenyl, C₂₋₈ alkynyl, 3- to 10-membered carbocyclyl, 4- to 10-membered heterocyclyl, 3- to 10-membered carbocyclyloxy or 4- to 10-membered heterocyclyloxy, k is selected from 0, 1, 2, 3, 4 or 5, and j is selected from 0, 1 or 2. The alkyl, k, j, R¹⁹ and R^{19a} herein are as defined above.

The "alkylene" refers to a linear or branched divalent saturated hydrocarbon group, including -(CH2)v- (v is an integer from 1 to 10), and examples of alkylene include, but are not limited to, methylene, ethylene, propylene and butylene. The alkylene can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxylalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)j-R¹⁹, -O-C(=O)-O-R¹⁹ or -NR¹⁹R^{19a}. When the number of substituents in the alkylene is greater than or equal to 2, the substituents may be fused together to form a cyclic structure. The "alkylene" herein is as defined above.

The "alkoxy" refers to a monovalent group of O-alkyl, wherein the alkyl is as defined herein, and examples of alkoxy include, but are not limited to methoxy, ethoxy, 1-propoxy, 2-propoxy, 1-butoxy, 2-methyl-1-propoxy, 2-butoxy, 2-methyl-2-propoxy, 1-pentyloxy, 2-pentyloxy, 3-pentyloxy, 2-methyl-2-butoxy, 3-methyl-2-butoxy, 3-methyl-1-butoxy and 2-methyl-1-butoxy.

The "alkenyl" refers to a linear or branched monovalent unsaturated hydrocarbon group having at least 1, usually 1, 2 or 3 carbon-carbon double bonds, in which the main chain comprises 2 to 10 carbon atoms, further preferably 2 to 6 carbon atoms, and more preferably 2 to 4 carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene and 1,4-hexadiene. The alkenyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxylalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. The "alkenyl" herein is as defined above.

The "alkynyl" refers to a linear or branched monovalent unsaturated hydrocarbon group having at least 1, usually 1, 2 or 3 carbon-carbon triple bonds, in which the main chain comprises 2 to 10 carbon atoms, further preferably 2 to 6 carbon atoms, and more preferably 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl and 4-decynyl. The alkynyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxylalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. The "alkynyl" herein is as defined above.

The "cycloalkyl" refers to a monovalent saturated carbocyclic hydrocarbon group, usually having from 3 to 10 carbon atoms, and non-limiting examples include cyclopropyl, cyclobutyl, cyclopentyl, cyclohexyl, cycloheptyl, etc. The cycloalkyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxylalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. The "cycloalkyl" herein is as defined above.

The "carbocycle" refers to a saturated or unsaturated aromatic ring or non-aromatic ring, and the aromatic ring or non-aromatic ring can be a 3- to 10-membered monocyclic ring, 4- to 12-membered bicyclic ring or 10- to 15-membered tricyclic ring system. Carbocyclyl can be linked to a bridged ring or spiro ring. Non-limiting examples include cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, cyclohexadienyl, cycloheptyl, cyclooctyl, cyclononyl, cyclodecyl, cycloundecyl, cyclododecyl, phenyl or naphthyl. The carbocyclyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxylalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. The "carbocycle" herein is as defined above.

The "heterocycle" refers to a saturated or unsaturated aromatic ring or non-aromatic ring; the aromatic ring or non-aromatic ring can be a 3- to 10-membered monocyclic ring, 4- to 12-membered bicyclic ring or 10- to 15-membered tricyclic ring system, and contains 1 to 4 heteroatoms selected from N, O or S, preferably 3-to 8-membered heterocyclyl; and the optionally substituted N and S in the heterocyclyl can be oxidized into various oxidation states. Heterocyclyl can be linked to a heteroatom or a carbon atom, and can be linked to a bridged ring or a spiro ring. Non-limiting examples include epoxyethyl, epoxypropyl, azacyclopropyl, oxacyclobutyl, azacyclobutyl, thiacyclobutyl, 1,3-dioxolanyl, 1,4-dioxolanyl, 1,3-dioxanyl, azacycloheptyl, oxacycloheptyl, thiacycloheptyl, oxazepinyl, diazepinyl, thiazepinyl, pyridyl, piperidinyl, homopiperidinyl, furyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, oxathianyl, dihydrofuranyl, dihydropyranyl, dithiocyclopentyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuryl, 2-pyrrolinyl, 3-pyrrolinyl, dihydroindolyl, 2H-pyranyl, 4H-pyranyl, dioxacyclohexyl, 1,3-dioxoclopentyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclohexyl, 3-azabicycloheptyl, azabicyclohexyl, 3H-indolylquinazinyl, N-pyridyl urea, 1,1-dioxothiomorpholinyl, azabicyclooctanyl, azabicyclononanyl, oxatricyclododecyl, azaadamantyl and oxaspiroheptanyl. The heterocyclyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxylalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, - (CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-OR¹⁹ or -NR¹⁹R^{19a}. The "heterocycle" herein is as defined above.

The term "optional" or "optionally" refers to that the event or circumstance subsequently described may but not necessarily occur, and the description includes the occasions where the events or circumstances occur or do not occur. For example, the "alkyl optionally substituted with F" means that the alkyl may but not necessarily be substituted with F, and the description includes the case where the alkyl is substituted with F and the case where the alkyl is not substituted with F.

The "pharmaceutical composition" represents a mixture of one or more compounds described herein or a physiologically/pharmaceutically acceptable salt thereof or a stereoisomer, solvate, pharmaceutically acceptable salt or eutectic thereof and other components comprising physiologically/pharmaceutically acceptable carriers and/or excipients.

The "stereoisomer" refers to an isomer produced as a result of different spatial arrangement of atoms in molecules, including cis-trans isomers, enantiomers and conformational isomers.

The "effective dose" refers to an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

The "solvate" refers to the compounds of the present invention or the salts thereof, and also includes stoichiometric or non-stoichiometric solvents bound by intermolecular non-covalent forces. When the solvent is water, the solvate is a hydrate.

### Brief Description of the Drawings

Figure 1 shows the inhibition ratio of compound 1 on compound 48/80-induced scratching behaviour in mice.
Figure 2 shows the fitting curve of the inhibition ratio (ED₅₀) of compound 1 on compound 48/80-induced scratching behaviour in mice.
Figure 3 shows the inhibition ratio of compound 1 on serotonin hydrochloride-induced scratching behaviour in rats.
Figure 4 shows the fitting curve of the inhibition ratio (ED₅₀) of compound 1 on serotonin hydrochloride-induced scratching behaviour in rats.
Figure 5 shows the inhibition ratio of compound 1 administered by nasal drip on compound 48/80-induced scratching behaviour in mice.
Figure 6 shows the fitting curve of the inhibition ratio (ED₅₀) of compound 1 administered by nasal drip on compound 48/80-induced scratching behaviour in mice.

### Detailed Description of Embodiments

The technical solutions of the present invention will be described in detail below in conjunction with the drawings and examples, but the scope of protection of the present invention includes but is not limited thereto.

### Synthesis example:

**Compound 1:** (2R)-N-[(1R)-1-(2-acetyl-2,7-diazaspiro[3.5]nonane-7-carbonyl)-5-amino-pentyl]-2-[[ (2R)-2- [[ (2R)-2-amino-3-phenylpropionyl]amino] -3-phenylpropionyl]amino]-4-methyl-pentanamide (CAS number: 2269511-95-5) was prepared according to the method described in WO 2019015644.

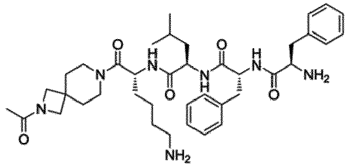

### Biological test examples

### Test example 1: Study on antipruritic effects of compound 1 in mouse models with pruritus induced by compound 48/80

ICR mice (males) from Beijing Vital River Laboratory Animal Technology Co., Ltd. were randomly grouped, and then administered intravenously with different doses of compound 1 or normal saline. After 15 min, 50 µg of compound 48/80 (Sigma, Cat#: C2313) was injected subcutaneously into the nape of the mice, and the times of scratching of mice within 30 min after injection of compound 48/80 was immediately recorded. The obtained data were processed according to the formula [(times of scratching in solvent control group - times of scratching in administration group)/times of scratching in solvent control group × 100] to calculate the inhibition ratio on scratching. Statistical analysis was performed with Graphpad Prism 8 software, and fitting was performed to obtain the ED₅₀ value.

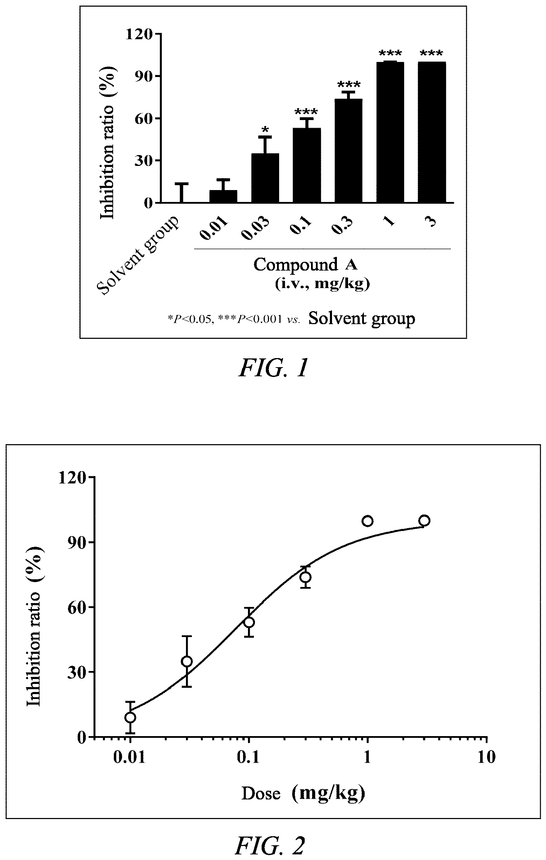

The results were as shown in Figures 1 and 2. Compound 1 inhibited the compound 48/80-induced scratching behaviour in mice in a dose-dependent manner, and the antipruritic effective dose and ED₅₀ value were 0.03 mg/kg and 0.09 mg/kg, respectively. Therefore, compound 1 had significant pharmacodynamic activity in mouse models with pruritus induced by compound 48/80.

### Test example 2: Study on antipruritic effects of single administration of compound 1 in rat models with pruritus induced by serotonin hydrochloride

SD rats (males) from Beijing Vital River Laboratory Animal Technology Co., Ltd. were randomly grouped, and then administered intravenously with different doses of compound 1 or normal saline. After 15 min, 10 µL of 2% serotonin hydrochloride (Sigma) was injected intradermally into the nape of the rats, and the times of scratching of rats within 60 min after injection of serotonin hydrochloride was immediately recorded. The obtained data were processed according to the formula [(times of scratching in solvent control group - times of scratching in administration group)/times of scratching in solvent control group × 100] to calculate the inhibition ratio on scratching. Statistical analysis was performed with Graphpad Prism 8 software, and fitting was performed to obtain the ED₅₀ value.

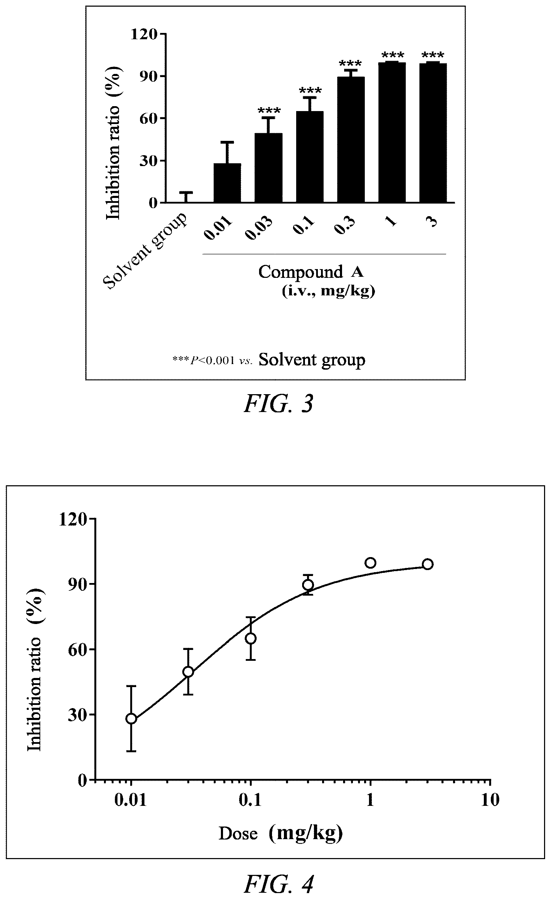

The results were as shown in Figures 3 and 4. Compound 1 inhibited the serotonin hydrochloride-induced scratching behaviour in rats in a dose-dependent manner, and the antipruritic effective dose and ED₅₀ value were both 0.03 mg/kg. Therefore, compound 1 had significant pharmacodynamic activity in rat models with pruritus induced by serotonin hydrochloride.

### Test example 3: Study on antipruritic effects of compound 1 administered by nasal drip in mouse models with pruritus induced by compound 48/80

ICR mice (males) from Beijing Vital River Laboratory Animal Technology Co., Ltd. were randomly grouped, and then different doses of compound 1 or normal saline were administered by nasal drip. After 15 min, 50 µg of compound 48/80 (Sigma, Cat#: C2313) was injected subcutaneously into the nape of the mice, and the times of scratching of mice within 30 min after injection of compound 48/80 was immediately recorded. The obtained data were processed according to the formula [(times of scratching in solvent control group - times of scratching in administration group)/times of scratching in solvent control group × 100] to calculate the inhibition ratio on scratching. Statistical analysis was performed with Graphpad Prism 8 software, and fitting was performed to obtain the ED₅₀ value.

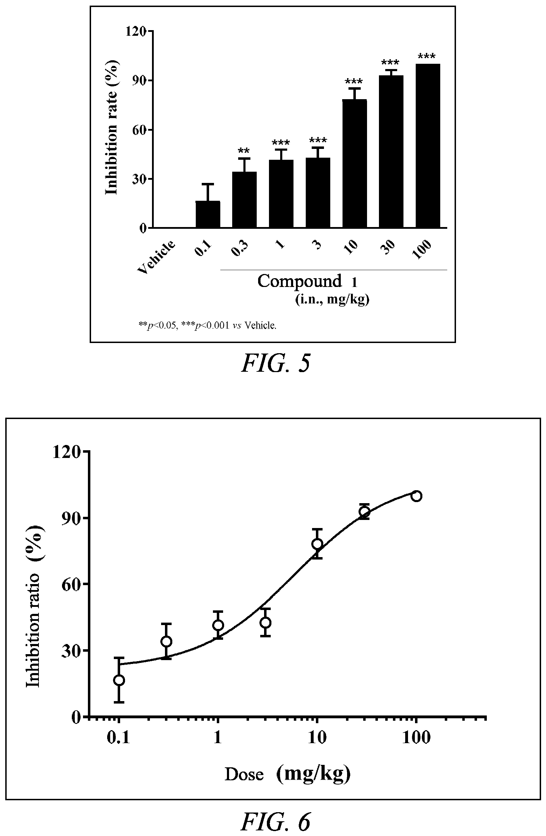

The results were as shown in Figures 5 and 6. Compound 1 inhibited the compound 48/80-induced scratching behaviour in mice in a dose-dependent manner, and the antipruritic effective dose and ED₅₀ value were 0.3 mg/kg and 2.72 mg/kg, respectively. Therefore, compound 1 administered by nasal drip had significant pharmacodynamic activity in mouse models with pruritus induced by compound 48/80. **Test example 4: Clinical trials**

### Test example 4-1 Multi-centre, randomized, double-blind, placebo-controlled phase II clinical study for safety, pharmacokinetics and efficacy in haemodialysis subjects

For compound 1, a multi-centre, randomized, double-blind, placebo-controlled phase II clinical trial for evaluating the safety, pharmacokinetics and efficacy of compound 1 injection in haemodialysis subjects was performed. This study was divided into two phases.

Phase I was a dose-escalation, randomized, double-blind, placebo-controlled study of multiple intravenous administration within 1 week in haemodialysis subjects, in which 40 haemodialysis subjects were scheduled to be enrolled into 4 dose groups: 0.05 µg/kg, 0.15 µg/kg, 0.30 µg/kg and 0.80 µg/kg, with 10 subjects in each group (including 2 subjects in placebo group), and the experimental drug was administered 3 times in each dose group on D1, D3 and D5, respectively.

Phase II was a randomized, double-blind, placebo-controlled study of administration for 12 weeks in haemodialysis subjects with moderate or more severe pruritus. According to the results of phase I study, two dose groups (0.3 µg/kg and 0.6 µg/kg) and a placebo group as control were selected for phase II study, in which 90 haemodialysis subjects with moderate or more severe pruritus were scheduled to be enrolled and assigned to the above-mentioned three groups according to a ratio of 1 : 1 : 1, with 30 subjects scheduled to be enrolled in each group, and compound 1 injection or placebo was administered, respectively.

In phase I, after the treatment in each group, the NRS scores all decreased compared to baseline, and the total score of Skindex-16 scale decreased. In phase II, after the treatment in each group, the NRS scores all decreased compared to baseline, and the scores that decreased compared to baseline showed an increase over time; and after the treatment in each group, the total score and dimension scores of Skindex-16 scale, and the total score and dimension scores of 5-D scale all decreased compared to baseline.

The efficacy results showed that in patients with maintenance haemodialysis, after the administration of compound 1 multiple times a week for consecutive 12 weeks, the pruritus symptoms in patients with chronic kidney diseases could be effectively relieved, while the overall quality of life was improved, wherein the 0.30 µg/kg group showed more significant improvement compared to the placebo group and had a good maintenance of therapeutic effects after discontinuation. The safety results showed that in patients with maintenance haemodialysis, compound 1 was generally safe at the dose of 0.05 µg/kg to 0.80 µg/kg, with no serious adverse events related to the experimental drug and no obvious withdrawal tendency after discontinuation.

### Test example 4-2: Multi-centre, randomized, double-blind, placebo-controlled phase III clinical trial for efficacy and safety on chronic kidney disease-related pruritus in patients with maintenance haemodialysis

For compound 1, a multi-centre, randomized, double-blind, placebo-controlled phase III clinical trial for evaluating the efficacy and safety of compound 1 injection on chronic kidney disease-related pruritus in patients with maintenance haemodialysis was performed.

This study included a double-blind phase and an open-label phase. The double-blind phase included a double-blind screening phase (4 weeks, including a 1-week run-in period) and a double-blind treatment phase (12 weeks). The subjects eligible for enrolment were randomly assigned to either an experimental group (0.3 µg/kg) or a control group (placebo) according to a ratio of 1 : 1. The open-label phase included an open-label screening phase (2 weeks; for subjects who completed the double-blind phase and entered directly the open-label phase, screening was conducted during the double-blind phase), an open-label treatment phase (52 weeks), and a follow-up phase (1 week). Subjects who had completed the double-blind phase study and met protocol requirements or other subjects who met the inclusion criteria and did not meet exclusion criteria for the open-label phase could voluntarily enter the open-label phase study, and all subjects received treatment with compound 1 at 0.3 µg/kg. During the double-blind treatment phase, either the compound 1 injection or a placebo was administered to subjects three times a week by group, and the experimental drug was administered after dialysis on D1 (the date of the first administration of the experimental drug in a week was defined as D1 of that week), D3 (+2) and D5 (+2) for consecutive 12 weeks. During the open-label treatment phase, the compound 1 injection was administered to all subjects three times a week for consecutive 52 weeks. In the study, approximately 544 subjects were scheduled to be enrolled, with approximately 272 subjects in the experimental group and approximately 272 subjects in the control group.

From the first administration in the double-blind phase to the end of the double-blind treatment phase, the assessment of the intensity of the most severe pruritus (worst itch numeric rating scale, WI-NRS) was performed daily. The assessment of quality of life (Skindex-16 scale, 5-D pruritus scale) was performed on the day of the first administration (defined as D1) and on the first visit day at weeks 5, 9, 11 and 13 after treatment. For the open-label phase: the assessment of quality of life (5-D pruritus scale) was performed on the first dialysis day at weeks 1, 5, 9, 13, 17, 25, 37 and 53 in the open-label treatment phase.

### Test example 4-3: Multi-centre, randomized, double-blind, placebo-controlled phase II clinical trial for efficacy, safety and pharmacokinetics in subjects with pruritus caused by liver diseases

For compound 1, a multi-centre, randomized, double-blind, placebo-controlled phase II clinical trial for evaluating the efficacy, safety and pharmacokinetics of compound 1 injection in subjects with pruritus caused by liver diseases was performed.

This study was a multi-centre, randomized, double-blind, placebo-controlled study. Approximately 90 liver disease subjects with moderate or more severe pruritus were scheduled to be enrolled and randomly assigned to three groups including two dose groups, 0.3 µg/kg and 0.6 µg/kg, and a placebo control group according to a ratio of 1 : 1 : 1, with approximately 30 subjects scheduled to be enrolled in each group. The subjects were administered twice in the morning and evening for 7-28 days. In the study, the subjects filled out the WI-NRS scale every morning and evening for the assessment of the intensity of pruritus. The assessment of quality of life was performed using the Skindex-16 scale on the first day of each week.

## Claims

1. Use of a compound represented by formula (I) or a stereoisomer, hydrate, metabolite, solvate, pharmaceutically acceptable salt or eutectic thereof, and a composition thereof in the preparation of a drug for preventing or treating pruritus, **characterized in that** the compound represented by formula (I) or the stereoisomer, hydrate, metabolite, solvate, pharmaceutically acceptable salt, eutectic or composition thereof is administered in an effective dose to a mammal, 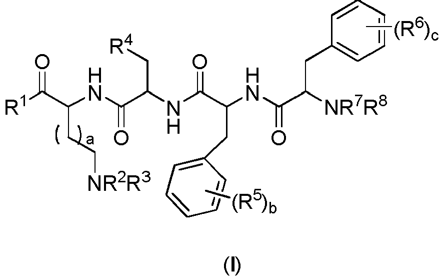 wherein
R₁ is selected from 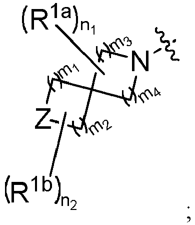
m1, m2, m3 and m4 are each independently selected from 0, 1, 2, 3 or 4; m1 and m2 are not both 0; m3 and m4 are not both 0;
n1 and n2 are each independently selected from 0, 1, 2, 3 or 4;
Z is selected from CR^{z1}R^{z2} or NR^{z3};
R^{z1} and R^{z2} are each independently selected from H, F, Cl, Br, I, OH, CF₃, nitro, C₁₋₆ alkyl, C₁₋₆ alkoxy, C₂₋₆ alkenyl, C₂₋₆ alkynyl, -C(=O)-C₁₋₆ alkyl, -(CH₂)q-C(=O)O-C₁₋₆ alkyl, -(CH₂)q-NR^{1e}R^{1f}, -(CH₂)q-COOH, -(CH₂)q-CONH₂, C₃₋₈ carbocyclyl, or 3- to 8-membered heterocyclyl, wherein the alkyl, alkoxy, alkenyl, alkynyl, carbocyclyl or heterocyclyl is optionally further substituted with 0-5 substituents selected from F, Cl, Br, I, OH, CF₃, =O, carboxyl, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, the heterocyclyl contains 1 to 3 heteroatoms optionally selected from N, O or S, and when the heteroatom is selected from S, the heterocyclyl is optionally further S, S=O or S(=O)₂;
R^{1e} and R^{1f} are each independently selected from H, C₁₋₆ alkyl, -C(=O)O-C₁₋₆ alkyl, -C(=O)O-(CH₂)q-C₃₋₈ carbocyclyl or -C(=O)O-(CH₂)q-3- to 8-membered heterocyclyl, wherein the alkyl, carbocyclyl or heterocyclyl is optionally further substituted with 0-5 substituents selected from F, Cl, Br, I, OH, CF₃, cyano, nitro, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
or R^{z1} and R^{z2} together with the carbon atom to which they are attached form a 3- to 10-membered nitrogen-containing heterocyclic ring, wherein the ring is optionally further substituted with a substituent selected from F, Cl, Br, I, OH, CF₃, cyano, nitro, =O, C₁₋₆ alkyl, C₁₋₆ alkoxy, C₂₋₆ alkenyl, C₂₋₆ alkynyl, C.carbocyclyl or 3- to 8-membered heterocyclyl;
R^{1a} and R^{1b} are each independently selected from F, CF₃, NH₂, C₁₋₆ alkyl, C₂₋₆ alkenyl, C₂₋₆ alkynyl or 3- to 8-membered heterocyclyl, wherein the alkyl, alkenyl, alkynyl or heterocyclyl is optionally further substituted with 0-5 substituents selected from F, Cl, Br, I, OH, CF₃, nitro, cyano, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{z3} is independently selected from H, -C(=O)-C₁₋₆ alkyl, -C(=O)O-C₁₋₆ alkyl, - C(=O)-C₃₋₈ carbocyclyl, -C(=O)O-C₃₋₈ carbocyclyl, -C(=O)O-(3- to 8-membered heterocyclyl), -S(=O)p-C₁₋₆ alkyl, -S(=O)p-C₃₋₈ carbocyclyl, -S(=O)p-(3- to 8-membered heterocyclyl), -C(=O)NR¹⁹R^{1h}, -S(=O)p-NR¹ⁱR^{1j} or 3- to 8-membered heterocyclyl, wherein the alkyl, carbocyclyl or heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{1g}, R^{1h}, R^{1i'} and R^{1j} are each independently selected from H or C₁₋₆ alkyl;
or R^{1g} and R^{1h} together with the nitrogen atom to which they are attached form a 3- to 10-membered heterocyclic ring, wherein the ring is optionally further substituted with a substituent selected from F, Cl, Br, I, OH, CF₃, nitro, cyano, amino, C₁₋₆ alkyl, C₁₋₆ alkoxy, C₂₋₆ alkenyl, C₂₋₆ alkynyl or -S(=O)p-C₁₋₆ alkyl, and the heterocyclyl contains 1 to 3 heteroatoms selected from N, O or S;
q is selected from 0, 1, 2, 3 or 4;
p is selected from 0, 1 or 2;
a is selected from 0, 1, 2 or 3;
R⁴ is independently selected from H, C₁₋₆ alkyl, C₂₋₆ alkenyl, C₂₋₆ alkynyl or - (CH₂)q-C₃₋₈ carbocyclyl, wherein the alkyl, alkenyl, alkynyl or carbocyclyl is optionally further substituted with 0-5 substituents selected from F, Cl, Br, I, OH, CN, CF₃, NO₂, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
R², R³, R⁷ and R⁸ are each independently selected from H, C₁₋₆ alkyl, -C(=O)O-C₁₋₄ alkyl, -C(=O)O-(CH₂)q-C₃₋₈ carbocyclyl, -C(=O)O-(CH₂)q-3- to 8-membered heterocyclyl or 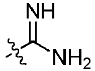 , wherein the alkyl, carbocyclyl or heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
b is selected from 0, 1, 2, 3, 4 or 5;
c is selected from 0, 1, 2, 3, 4 or 5;
R⁵ and R⁶ are each independently selected from F, Cl, Br, I, OH, CN, CF₃, cyano, nitro, C₁₋₄ alkyl, -OR^{5a}, -C(O)OR^{5b}, -SR^{5c}, -S(O)R^{5d}, -S(O)₂R^{5e} or -NR^{5f}R^{5g};
R^{5a}, R^{5b}, R^{5c}, R^{5d}, R^{5e}, R^{5f} and R^{5g} are each independently selected from H or C₁₋₄ alkyl;
or R^{5f} and R^{5g} together with the nitrogen atom to which they are attached form a 5- to 6-membered heterocyclic ring, wherein the heterocyclyl contains 1 to 3 heteroatoms optionally selected from N, O or S.

2. The use according to claim 1, wherein the compound represented by formula (I) is selected from a compound represented by formula (II): 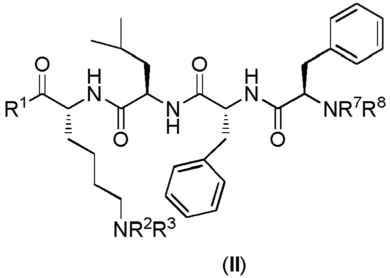 wherein
R₁ is selected from 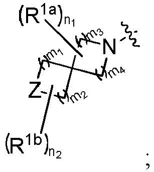
m1, m2, m3 and m4 are each independently selected from 0, 1 or 2; m1 and m2 are not both 0; m3 and m4 are not both 0;
n1 and n2 are each independently selected from 0 or 2;
Z is selected from CR^{z1}R^{z2} or NR^{z3};
R^{1a} and R^{1b} are independently selected from F or NH₂;
R^{z1} and R^{z2} are each independently selected from H, carboxyl, 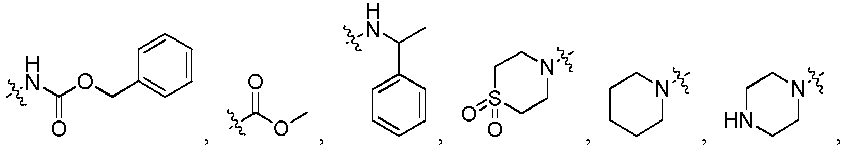 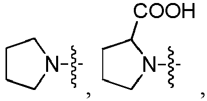 amino, -CH₂NH₂ or 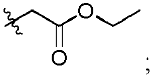 or R^{z1} and R^{z2} can together with the carbon atom to which they are attached form a lactam 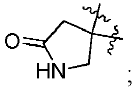
R^{z3} is independently selected from H, -C(=O)-C₁₋₄ alkyl, -C(=O)O-C₁₋₄ alkyl, - C(=O)-C₃₋₆ carbocyclyl, -C(=O)O-C₃₋₆ carbocyclyl, -S(=O)p-C₁₋₄ alkyl, -S(=O)p-C₃₋₆ carbocyclyl, -C(=O)NR^{1g}R^{1h}, -S(=O)p-NR¹ⁱR^{1j} or 3- to 6-membered heterocyclyl, wherein the alkyl, carbocyclyl or heterocyclyl is optionally further substituted with 0-5 substituents selected from F, Cl, Br, I, OH, CF₃, nitro, cyano, amino, methyl, ethyl, methoxy, ethoxy, cyclopropyl or phenyl, and the heterocyclyl contains 1 to 3 heteroatoms optionally selected from N, O or S;
R^{1g}, R^{1h}, R^{1i'} and R^{1j} are each independently selected from H or C₁₋₄ alkyl;
or R^{1g} and R^{1h} together with the nitrogen atom to which they are attached form a 4- to 6-membered heterocyclic ring, wherein the ring is optionally further substituted with a substituent selected from F, CF₃, methyl, methoxy or -S(=O)p-C₁₋₄ alkyl, and the heterocyclyl contains 1 to 3 heteroatoms selected from N, O or S;
p is selected from 2;
R², R³, R⁷ and R⁸ are each independently selected from H, methyl or C(=O)O-tert-butyl.

3. The use according to claim 2, wherein the compound represented by formula (II) is selected from a compound represented by structural formula (A) as follows:

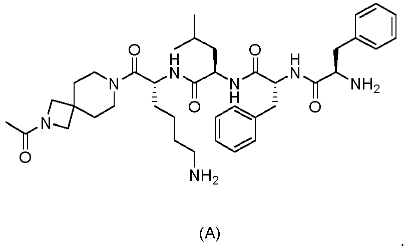

4. The use according to claim 3, **characterized in that** the compound represented by structural formula (A) or a stereoisomer, hydrate, metabolite, solvate, pharmaceutically acceptable salt, eutectic or composition thereof is administered in an effective dose to a mammal.

5. The use according to claim 4, wherein the pharmaceutically acceptable salt is selected from propionate, mesylate, acetate, citrate, D-tartrate, besylate, phosphate, aspartate, L-tartrate, maleate, fumarate, benzoate, lactate, hydrochloride, formate, hydrobromide, sulphate, nitrate, phosphate, trifluoroacetate, succinate, mandelate, malonate, malate, 2-hydroxypropionate, oxalate, glycolate, salicylate, a citric acid salt, glutamate, cinnamate, p-toluenesulfonate, benzenesulfonate, ethanesulfonate or triflate.

6. The use according to either claim 4 or 5, wherein the pruritus is selected from pruritus caused by a disease, pruritus caused by a drug, or kidney disease-related pruritus.

7. The use according to claim 6, wherein the disease includes an inflammatory or non-inflammatory skin disease, and a peripheral or systemic disease.

8. The use according to claim 7, wherein
the inflammatory or non-inflammatory skin disease is selected from atopic dermatitis, neurodermatitis, contact dermatitis, seborrheic dermatitis, prurigo and senile dermatosis;
the peripheral or systemic disease is selected from a liver disease, a kidney disease, an autoimmune disease or peripheral neuropathy;
the pruritus caused by a drug is selected from pruritus caused by obeticholic acid, an antibiotic, an angiotensin converting enzyme inhibitor, a xanthine oxidase inhibitor or morphine.

9. The use according to claim 8, wherein
the liver disease is selected from an autoimmune liver disease (e.g., primary biliary cholangitis, primary sclerosing cholangitis or autoimmune hepatitis), viral hepatitis (e.g., hepatitis A, hepatitis B, hepatitis C, hepatitis D or hepatitis E), tumour (e.g., hepatocellular carcinoma or cholangiocarcinoma), liver cirrhosis, alcoholic liver disease, non-alcoholic fatty liver disease, drug-induced hepatitis, cholestasis (e.g., benign recurrent intrahepatic cholestasis, progressive familial intrahepatic cholestasis or intrahepatic cholestasis of pregnancy) or jaundice;
the kidney disease is selected from nephritis, a chronic kidney disease, uraemia or diabetic nephropathy;
the autoimmune disease is selected from scleroderma or Sjogren syndrome;
the peripheral neuropathy is selected from brachioradial pruritus, notalgia paresthetica or small fibre neuropathy.

10. The use according to any one of claims 3 to 9, wherein in terms of the compound represented by structural formula (A), the amount of the compound is 1-1000 µg/day.

11. The use according to claim 10, wherein the compound represented by structural formula (A) or the stereoisomer, hydrate, metabolite, solvate, pharmaceutically acceptable salt, eutectic or composition thereof is administered at an interval selected from once a day, twice a day, three times a day, once a week, twice a week, three times a week or once every other day.

12. The use according to claim 10, wherein the compound represented by structural formula (A) or the stereoisomer, hydrate, metabolite, solvate, pharmaceutically acceptable salt, eutectic or composition thereof is administered via a route selected from oral route, injection, drip, transdermal route, sublingual route, parenteral route, intraperitoneal route, rectal route, buccal route, nasal drip, inhalation, local delivery, subcutaneous route, intra-adipose route, intra-articular route, intraperitoneal route or intrathecal route.

13. The use according to claim 10, wherein the compound represented by structural formula (A) or the stereoisomer, hydrate, metabolite, solvate, pharmaceutically acceptable salt, eutectic or composition thereof is administered via a route selected from oral route, intravenous injection, intravenous drip, intra-arterial injection, intramuscular injection, subcutaneous injection, intra-articular injection, intraperitoneal injection, intrathecal injection or nasal drip.

14. A method for treating pruritus, comprising administering an effective dose of the compound represented by formula (I) or the stereoisomer, hydrate, metabolite, solvate, pharmaceutically acceptable salt, eutectic or composition thereof to a mammal, wherein the effective dose is 1-1000 µg/day, preferably 2 µg-5 µg/day, 6 µg-15 µg/day, 12 µg-30 µg/day, 24 µg-60 µg/day, 32-80 µg/day, 4 µg-10 µg/day, 12 µg-30 µg/day, 24 µg-60 µg/day, 48 µg-120 µg/day, 64-160 µg/day, 6 µg-15 µg/day, 18 µg-45 µg/day, 36 µg-90 µg/day, 72 µg-180 µg/day or 96-240 µg/day, 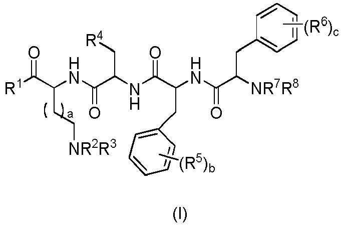 wherein the definition of each group is the same as that in claim 1.

15. The method according to claim 14, wherein the compound represented by formula (I) is selected from a compound represented by formula (II): 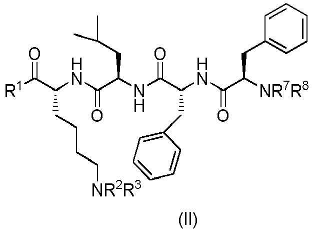 wherein the definition of each group is the same as that in claim 2.

16. The method according to claim 15, wherein the compound represented by formula (II) is selected from a compound represented by structural formula (A) as follows:

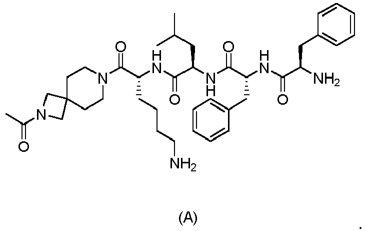

17. The method according to claim 16, comprising administering an effective dose of the compound represented by structural formula (A) or the stereoisomer, hydrate, metabolite, solvate, pharmaceutically acceptable salt, eutectic or composition thereof to a mammal.

18. The method according to claim 17, wherein the pharmaceutically acceptable salt is selected from propionate, mesylate, acetate, citrate, D-tartrate, besylate, phosphate, aspartate, L-tartrate, maleate, fumarate, benzoate, lactate, hydrochloride, formate, hydrobromide, sulphate, nitrate, phosphate, trifluoroacetate, succinate, mandelate, malonate, malate, 2-hydroxypropionate, oxalate, glycolate, salicylate, a citric acid salt, glutamate, cinnamate, p-toluenesulfonate, benzenesulfonate, ethanesulfonate or triflate.

19. The method according to either claim 16 or 17, wherein the pruritus is selected from pruritus caused by a disease, pruritus caused by a drug, or kidney disease-related pruritus.

20. The method according to claim 19, wherein the disease includes an inflammatory or non-inflammatory skin disease, and a peripheral or systemic disease.

21. The method according to claim 20, wherein the inflammatory or non-inflammatory skin disease is selected from atopic dermatitis, neurodermatitis, contact dermatitis, seborrheic dermatitis, prurigo and senile dermatosis;
the peripheral or systemic disease is selected from a liver disease, a kidney disease, an autoimmune disease or peripheral neuropathy;
the pruritus caused by a drug is selected from pruritus caused by obeticholic acid, an antibiotic, an angiotensin converting enzyme inhibitor, a xanthine oxidase inhibitor or morphine.

22. The method according to claim 21, wherein the liver disease is selected from an autoimmune liver disease (e.g., primary biliary cholangitis, primary sclerosing cholangitis or autoimmune hepatitis), viral hepatitis (e.g., hepatitis A, hepatitis B, hepatitis C, hepatitis D or hepatitis E), tumour (e.g., hepatocellular carcinoma or cholangiocarcinoma), liver cirrhosis, alcoholic liver disease, non-alcoholic fatty liver disease, drug-induced hepatitis, cholestasis (e.g., benign recurrent intrahepatic cholestasis, progressive familial intrahepatic cholestasis or intrahepatic cholestasis of pregnancy) or jaundice;
the kidney disease is selected from nephritis, a chronic kidney disease, uraemia or diabetic nephropathy;
the autoimmune disease is selected from scleroderma or Sjogren syndrome;
the peripheral neuropathy is selected from brachioradial pruritus, notalgia paresthetica or small fibre neuropathy.

23. The method according to any one of claims 16 to 22, wherein the compound represented by structural formula (A) or the stereoisomer, hydrate, metabolite, solvate, pharmaceutically acceptable salt, eutectic or composition thereof is administered at an interval selected from once a day, twice a day, three times a day, once a week, twice a week, three times a week or once every other day.

24. The method according to any one of claims 16 to 22, wherein the compound represented by structural formula (A) or the stereoisomer, hydrate, metabolite, solvate, pharmaceutically acceptable salt, eutectic or composition thereof is administered via a route selected from oral route, injection, drip, transdermal route, sublingual route, parenteral route, intraperitoneal route, rectal route, buccal route, nasal drip, inhalation, local delivery, subcutaneous route, intra-adipose route, intra-articular route, intraperitoneal route or intrathecal route.

25. The method according to claim 24, wherein the compound represented by structural formula (A) or the stereoisomer, hydrate, metabolite, solvate, pharmaceutically acceptable salt, eutectic or composition thereof is administered via a route selected from oral route, intravenous injection, intravenous drip, intra-arterial injection, intramuscular injection, subcutaneous injection, intra-articular injection, intraperitoneal injection, intrathecal injection or nasal drip.
